# EUROPEAN PATENT APPLICATION

(11) **EP 2 755 155 A2**
(43) Date of publication of application: **16.07.2014**
(21) Application number: 14150378.9
(22) Date of filing: 07.01.2014
(51) Int. Cl.: G06F 19/00

(54) **Computer implemented method of collecting data relating to an adverse event relating to use of a substance**

(30) Priority: 11.01.2013 GB 201300517
(71) Applicant: MyMedsandMe Limited, Aylesbury HP18 0RA (GB)
(72) Inventor: Rut, Andrew, Foxton, CB22 6ST (GB); Gibbs, Trevor, London, W11 3LN (GB)
(74) Representative: Jones, Nicholas Michael

(57) **Abstract**

The invention provides a computer implemented method of collecting data relating to an adverse event relating to use of a substance, the method comprising:
detecting a user input relating to an identity of the substance or to an identity of the adverse event;
determining the identity of the substance or of the adverse event based on the user input; and
selectively displaying to the user at least one further request for information, wherein the content of the request displayed to the user is dependent on the identity of the substance or of the adverse event.
The user input may comprise at least partial completion of an auto-complete text field requesting the identity of the substance or adverse event, and the method may comprise the auto-complete text field displaying at least one potential substance or adverse event, for instance in a drop down menu.
A request for user input relating to an identity of the adverse event may be displayed and the request may comprise displaying a body map and information relating to at least one potential adverse effect associated with the body map or a part thereof.

## Description

### Field

The specification relates to collection of data generally and, in particular to the collection of data relating to an adverse event relating to use of a substance.

### Background

The ability to receive feedback from consumers is important to providers of various goods and services. The feedback allows the provider to gauge whether any changes need to be made to their product, advertising or labelling to comply with government regulations or commercial factors.

For example, pharmaceutical companies seek information from users of their medical products regarding any adverse effect that may have been experienced. Conventionally, this has been done through submitting paper forms to the manufacturer or via a telephone interview. Alternatively, a doctor or other medical professional can submit details on their patient's behalf.

With the advent of the internet there has developed a possibility for a user or medical professional to submit details of an adverse effect via an online questionnaire. However, the information gathered in this way is often deficient because the fields requesting information are unresponsive to information entered in a previous field. In other words, there is no means for presenting additional, follow-up, questions to the user in response to an answer they have already given.

As such the online experience of the user does not match the experience of speaking directly to the provider or to a medical professional directly.

### Summary

A first aspect of the invention provides a computer implemented method of collecting data relating to an adverse event relating to use of a substance, the method comprising:
detecting a user input relating to an identity of the substance or to an identity of the adverse event;
determining the identity of the substance or of the adverse event based on the user input; and
selectively displaying to the user at least one further request for information, wherein the content of the request displayed to the user is dependent on the identity of the substance or of the adverse event.

The user input may comprise at least partial completion of an auto-complete text field requesting the identity of the substance or adverse event, and the method may comprise the auto-complete text field displaying at least one potential substance or adverse event, for instance in a drop down menu.

A request for user input relating to an identity of the adverse event may be displayed and the request may comprise displaying a body map and information relating to at least one potential adverse effect associated with the body map or a part thereof. Determining an identity of the adverse effect may comprise detecting a user selection of a part of the displayed body map and a user selection of information relating to an adverse effect from the at least one displayed potential adverse effect associated with the selected part of the body map.

The at least one potential adverse effect may be a medical side effect which may be displayed as a recognised medical term or as a colloquial term associated with a recognised medical term in MedDRA.

The method may comprise requesting a further user input comprising at least partial completion of an auto-complete text field requesting the identity of one of either a medical condition or a medical investigation, comprising the auto-complete text field displaying, respectively, at least one potential medical condition or medical investigation in a drop down menu. The method may comprise the auto-complete text field displaying, respectively, the at least one potential medical condition or medical investigation in the drop down menu with most frequently reported terms appearing first, and optionally updating a record of frequencies at which terms are reported. The method may comprise the auto-complete text field displaying, respectively, the at least one potential medical condition or medical investigation in the drop down menu with terms unrelated to adverse events filtered out.

The method may comprise exporting collected data in a structured format to a client database.

The method may comprise responding to detection of entry of one of a list of predetermined specific product names and/or events by providing a response comprising feedback or one or more additional questions.

The method may comprise displaying a list of selectable potential adverse event scenarios to the user.

A second aspect of the invention comprises a computer implemented method of collecting data relating to an adverse event, the method comprising:
detecting a user input relating to an identity of a substance;
determining the identity of the substance based on the user input;
selectively displaying to the user at least one potential medical indication of the identified substance based on the identity of the substance; and
detecting a second user input to select the indication of the substance from the at least one displayed potential indication.

The user input may comprise at least partial completion of an auto-complete text field requesting the identity of the substance, and the auto-complete text field may be configured to display at least one potential substance, for instance in a drop down menu.

The method may comprise:
detecting a user input relating to an identity of an adverse event;
determining the identity of the adverse event based on the user input; and
selectively displaying to the user at least one further request for information, wherein the content of the request displayed to the user is dependent on the identity of the substance or of the adverse event.

A request for user input relating to an identity of the adverse event may be displayed and the request may comprise displaying a body map and information relating to at least one potential adverse effect associated with the body map or a part thereof.

Determining an identity of the adverse effect may comprise detecting a user selection of a part of the displayed body map and a user selection of information relating to an adverse effect from the at least one displayed potential adverse effect associated with the selected part of the body map.

The at least one potential adverse effect may be a medical side effect which may be displayed as a recognised medical term or as a colloquial term associated with a recognised medical term coded in MedDRA displayed as a recognised medical term or as a colloquial term associated with a recognised medical term coded in MedDRA.

A further user input may be requested comprising at least partial completion of an auto-complete text field requesting the identity of one of either a medical condition or a medical investigation, and the method may comprise the auto-complete text field displaying, respectively, at least one potential medical condition or medical investigation in a drop down menu.

The method may comprise the auto-complete text field displaying, respectively, the at least one potential medical condition in the drop down menu with each of the product indications represented as a coded MedDRA term.

The method may comprise the auto-complete text field displaying, respectively, the at least one potential medical condition in the drop down menu represented in colloquial language that is linked to a medical term in MedDRA.

The method may comprise exporting collected data in a structured format to a client database.

The method may comprise responding to detection of entry of one of a list of predetermined specific product names and/or events by providing a response comprising feedback or one or more additional questions.

A third aspect of the invention provides a computer implemented method of collecting information relating to an adverse event, the method comprising:
displaying an interactive map representing a human body comprising a plurality of selectable elements;
detecting a user selection of one of the selectable elements;
in response to the user selection, displaying at least one potential adverse effect associated with the selected element; and
detecting a user selection of the or one of the displayed potential adverse effects.

The method may comprise:
displaying an exploded view of the selected element in response to the user selection, the exploded view of the selected element comprising a plurality of selectable subelements;
detecting a user selection of one of the selectable sub-elements;
in response to the user selection, displaying at least one potential adverse effect associated with the selected subelement; and
detecting a user selection of the or one of the displayed potential adverse effects associated with the selected subelement.

The invention also provides computer program instructions that, when executed, perform the method above.

A fourth aspect of the invention provides apparatus for collecting data relating to an adverse event relating to use of a substance, the apparatus comprising:
means for detecting a user input relating to an identity of the substance or to an identity of the adverse event;
means for determining the identity of the substance or of the adverse event based on the user input; and
means for selectively displaying to the user at least one further request for information, wherein the content of the request displayed to the user may be dependent on the identity of the substance or of the adverse event.

The user input may comprise at least partial completion of an auto-complete text field requesting the identity of the substance or adverse event, and the auto-complete text field may be configured to display at least one potential substance or adverse event, for instance in a drop down menu.

A request for user input relating to an identity of the adverse event may be displayed and the request may comprise displaying a body map and information relating to at least one potential adverse effect associated with the body map or a part thereof. The means for determining an identity of the adverse effect may comprise means for detecting a user selection of a part of the displayed body map and a user selection of information relating to an adverse effect from the at least one displayed potential adverse effect associated with the selected part of the body map.

The at least one potential adverse effect may be displayed as a recognised medical term or as a colloquial term associated with a recognised medical term in MedDRA.

The apparatus may comprise means for requesting a further user input comprising at least partial completion of an auto-complete text field requesting the identity of one of either a medical condition or a medical investigation, comprising the auto-complete text field displaying, respectively, at least one potential medical condition or medical investigation in a drop down menu. The apparatus may comprise the auto-complete text field configured to display, respectively, the at least one potential medical condition or medical investigation in the drop down menu with most frequently reported terms appearing first, and optionally configured to update a record of frequencies at which terms are reported. The apparatus may comprise the auto-complete text field configured to display, respectively, the at least one potential medical condition or medical investigation in the drop down menu with terms unrelated to adverse events filtered out.

The apparatus may comprise exporting collected data in a structured format to a client database.

The apparatus may comprise means for responding to detection of entry of one of a list of predetermined specific product names and/or events by providing a response comprising feedback or one or more additional questions.

The apparatus may comprise means for displaying a list of selectable potential adverse event scenarios to the user.

A fifth aspect of the invention provides apparatus for collecting data relating to an adverse event, the apparatus comprising:
means for detecting a user input relating to an identity of a substance;
means for determining the identity of the substance based on the user input;
means for selectively displaying to the user at least one potential medical indication of the identified substance based on the identity of the substance; and
means for detecting a second user input to select the indication of the substance from the at least one displayed potential indication.

The user input may comprise at least partial completion of an auto-complete text field requesting the identity of the substance, and the auto-complete text field may be configured to display at least one potential substance, for instance in a drop down menu.

The apparatus may comprise:
means for detecting a user input relating to an identity of an adverse event;
means for determining the identity of the adverse event based on the user input; and
means for selectively displaying to the user at least one further request for information, wherein the content of the request displayed to the user may be dependent on the identity of the substance or of the adverse event.

A request for user input relating to an identity of the adverse event may be displayed and the request may comprise means for displaying a body map and information relating to at least one potential adverse effect associated with the body map or a part thereof.

The means for determining an identity of the adverse effect may comprise means for detecting a user selection of a part of the displayed body map and a user selection of information relating to an adverse effect from the at least one displayed potential adverse effect associated with the selected part of the body map.

The at least one potential adverse effect may be displayed as a recognised medical term or as a colloquial term associated with a recognised medical term coded in MedDRA.

A further user input may be requested comprising at least partial completion of an auto-complete text field requesting the identity of one of either a medical condition or a medical investigation, comprising the auto-complete text field comprising means for displaying, respectively, at least one potential medical condition or medical investigation in a drop down menu.

The apparatus may comprise the auto-complete text field comprising means for displaying, respectively, the at least one potential medical condition in the drop down menu with each of the product indications represented as a coded MedDRA term.

The apparatus may comprise the auto-complete text field comprising means for displaying, respectively, the at least one potential medical condition in the drop down menu represented in colloquial language that is linked to a medical term in MedDRA.

The apparatus may comprise means for exporting collected data in a structured format to a client database.

The apparatus may comprise means for responding to detection of entry of one of a list of predetermined specific product names and/or events by providing a response comprising feedback or one or more additional questions.

A sixth aspect of the invention provides apparatus for collecting information relating to an adverse event, the apparatus comprising:
means for displaying an interactive map representing a human body comprising a plurality of selectable elements;
means for detecting a user selection of one of the selectable elements;
means for, in response to the user selection, displaying at least one potential adverse effect associated with the selected element; and
means for detecting a user selection of the or one of the displayed potential adverse effects.

The apparatus may comprise:
means for displaying an exploded view of the selected element in response to the user selection, the exploded view of the selected element comprising a plurality of selectable subelements;
means for detecting a user selection of one of the selectable sub-elements;
means for, in response to the user selection, displaying at least one potential adverse effect associated with the selected subelement; and
means for detecting a user selection of the or one of the displayed potential adverse effects associated with the selected subelement.

A seventh aspect of the invention provides apparatus having at least one processor and at least one memory having computer-readable code stored thereon which when executed controls the at least one processor to collect data relating to an adverse event relating to use of a substance by:
detecting a user input relating to an identity of the substance or to an identity of the adverse event;
determining the identity of the substance or of the adverse event based on the user input; and
selectively displaying to the user at least one further request for information, wherein the content of the request displayed to the user may be dependent on the identity of the substance or of the adverse event.

An eighth aspect of the invention provides apparatus having at least one processor and at least one memory having computer-readable code stored thereon which when executed controls the at least one processor to collect data relating to an adverse event relating to use of a substance by:
detecting a user input relating to an identity of a substance;
determining the identity of the substance based on the user input;
selectively displaying to the user at least one potential medical indication of the identified substance based on the identity of the substance; and
detecting a second user input to select the indication of the substance from the at least one displayed potential indication.

A ninth aspect of the invention provides apparatus having at least one processor and at least one memory having computer-readable code stored thereon which when executed controls the at least one processor to collect data relating to an adverse event by:
displaying an interactive map representing a human body comprising a plurality of selectable elements;
detecting a user selection of one of the selectable elements;
in response to the user selection, displaying at least one potential adverse effect associated with the selected element; and
detecting a user selection of the or one of the displayed potential adverse effects.

A tenth aspect of the invention provides a non-transitory computer-readable storage medium having stored thereon computer-readable code, which, when executed by computing apparatus, causes the computing apparatus to collect data relating to an adverse event relating to use of a substance by:
detecting a user input relating to an identity of the substance or to an identity of the adverse event;
determining the identity of the substance or of the adverse event based on the user input; and
selectively displaying to the user at least one further request for information, wherein the content of the request displayed to the user may be dependent on the identity of the substance or of the adverse event.

An eleventh aspect of the invention provides a non-transitory computer-readable storage medium having stored thereon computer-readable code, which, when executed by computing apparatus, causes the computing apparatus to collect data relating to an adverse event relating to use of a substance by:
detecting a user input relating to an identity of a substance;
determining the identity of the substance based on the user input;
selectively displaying to the user at least one potential medical indication of the identified substance based on the identity of the substance; and
detecting a second user input to select the indication of the substance from the at least one displayed potential indication.

A twelfth aspect of the invention provides a non-transitory computer-readable storage medium having stored thereon computer-readable code, which, when executed by computing apparatus, causes the computing apparatus to collect data relating to an adverse event by:
displaying an interactive map representing a human body comprising a plurality of selectable elements;
detecting a user selection of one of the selectable elements;
in response to the user selection, displaying at least one potential adverse effect associated with the selected element; and
detecting a user selection of the or one of the displayed potential adverse effects.

### Brief Description of the Drawings

Embodiments of the present invention will now be described, by way of example only, with reference to the accompanying drawings, in which:
Figure 1 is a schematic diagram illustrating components of a system;
Figures 2 and 3 are screenshots of a user interface provided by the system shown in Figure 1;
Figure 4 is a flow chart illustrating an exemplary process according to one embodiment; and

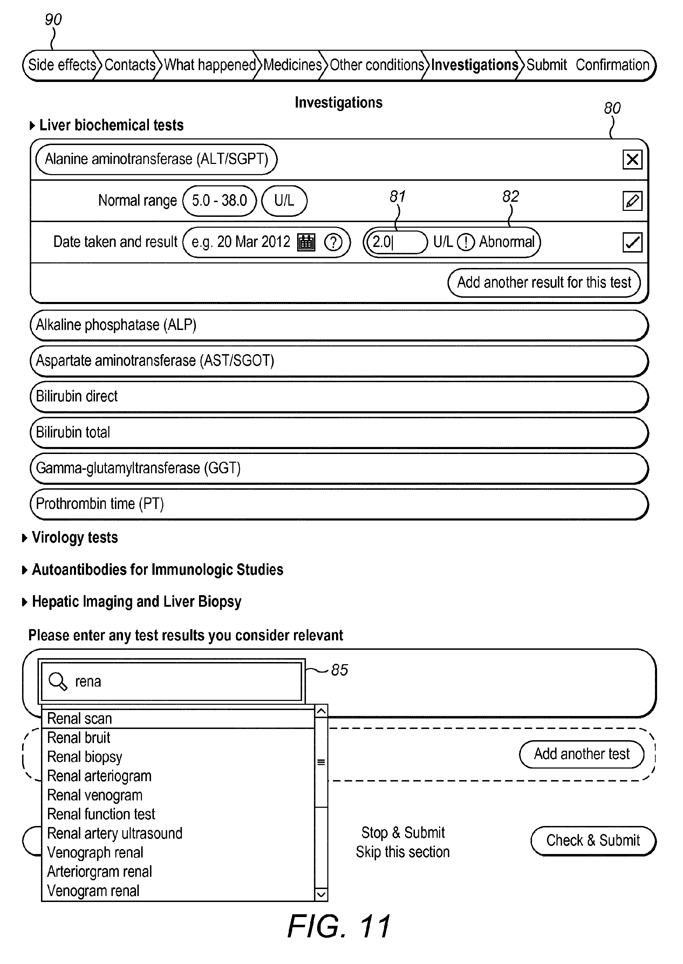

Figures 5-11 are screenshots of a user interface provided by the system shown in Figure 1.

### Detailed Description

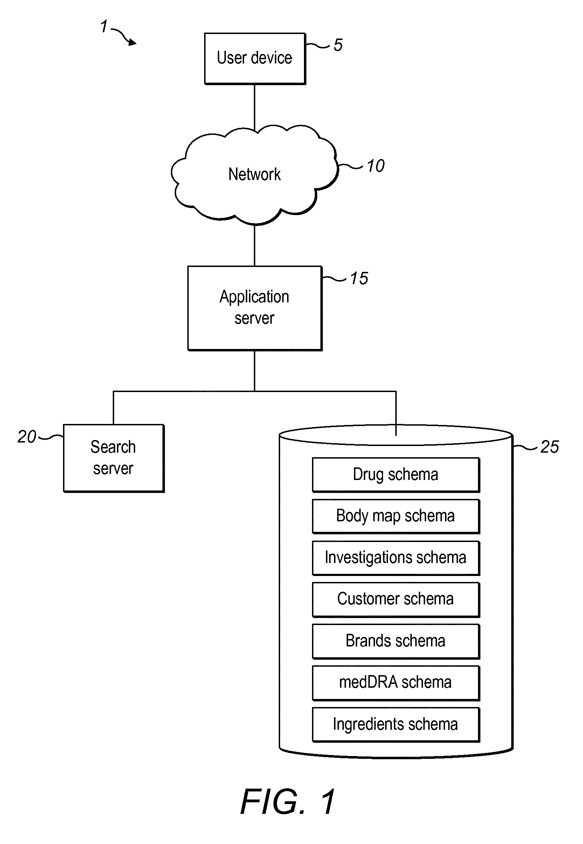

Figure 1 shows a system 1. The system 1 comprises a user device 5. The user device 5 may be a personal computer, tablet device, smartphone or any other computing device. The user device 5 may be provided with a processor, a memory, input hardware, output hardware and a network interface.

The memory may have an operating system, such as a Windows, Apple or Linux operating system, stored therein. Also stored in the memory may be applications such as a web browser.

The input hardware may take any suitable form and may comprise at least one of a keyboard, a mouse, an optical tracking ball, a touchpad and so forth to allow a user to input information to the user device 5. The output hardware may comprise a display screen, speakers and so forth so that information may be output to a user. The input hardware and output hardware may be integrated and may take the form of a touch sensitive display. The term 'user' as used herein signifies a person using the user device 5 to enter information about a side effect.

The network interface allows the user device 5 to access a network 10, for example the internet or a local area network. The network interface 5 may comprise a modem to allow access to the internet. Connection to the network 10 may be wireless or through a wired connection. A wireless network interface card may be provided. The user device 5 may be provided with a transceiver to allow access to a wireless router having a connection to the network 10 or to other devices in a local area network.

The processor is connected to and controls operation of the other components of the user device 5. The processor may execute software stored in the memory and/or control the user device 5 to execute instructions received over the network 10.

As stated above, the network 10 may be the internet. In this case, the reference numeral 10 should be understood as including elements such as routers and servers conventionally found in an internet architecture.

The system 1 also comprises an application server 15. The application server 15 comprises hardware and software components to allow the user device 5 to access the application. Server software used to allow access to the application may be Apache HTTP Server although any other suitable server software may be used. The application server 15 may use an application framework such as Ruby on Rails although the skilled person will understand that various other suitable application frameworks may be used in the alternative. The application framework may use a Model-View-Controller (MVC) architecture. The application server 15 may comprise a memory for storing application software and a processor for controlling the server. The application server 15 may be a single entity or it may comprise several entities that interact to perform the role of a server.

The application software stored in the application server 15 may provide the logic and routines that enables the application server 15 to perform the functionality described below. The application software may be pre-programmed into the application server 15. Alternatively, they may arrive at the application server 15 via an electromagnetic carrier signal or be copied from a physical entity such as a computer program product, a non-volatile electronic memory device (e.g. flash memory) or a record medium such as a CD-ROM or DVD. They may for instance be downloaded to the application server 15 from a server.

The processor may be any type of processing circuitry. For example, the processing circuitry may be a programmable processor that interprets computer program instructions and processes data. The processing circuitry may include plural programmable processors. Alternatively, the processing circuitry may be, for example, programmable hardware with embedded firmware. The processing circuitry or processor may be termed processing means.

The term 'memory' when used in this specification is intended to relate primarily to memory comprising both non-volatile memory and volatile memory unless the context implies otherwise, although the term may also cover one or more volatile memories only, one or more non-volatile memories only, or one or more volatile memories and one or more non-volatile memories. Examples of volatile memory include RAM, DRAM, SDRAM etc. Examples of non-volatile memory include ROM, PROM, EEPROM, flash memory, optical storage, magnetic storage, etc.

The application server 15 may be connected to a search server 20 and to a database 25. The connection between the application server 15, search server 20 and database 25 may be over the internet or any other suitable network connection. While shown in Figure 1 as separate entities it should be borne in mind that the application server 15, search server 20 and the database 25 may be integrated or otherwise arranged to allow the application to access data stored in the database 25 using a search function. In embodiments where a separate search server 20 is used the search server 20 may be an Elastic Search server although other search servers may be used. The search server 20 indexes data stored in the database 25 so that the data may be retrieved in response to a search request received at the application server 15 from the user device 5. Information stored in the database 25 may be organised into various schema. For example, information relating to drugs may be stored in a drugs schema, investigations information may be organised in an investigations schema and so forth. Organising stored information into schemas allows easier searching.

The application may be a web-based application that is accessible to a user device 5 using the hypertext transfer protocol (HTTP). Application content is thereby displayed by the user device 5, on a web browser, as a web page in hypertext mark-up language (HTML) or any other suitable language.

The web-based application takes the form of a series of pages displayed to the user on a user device. The user can enter information regarding a suspect medicine and details of an adverse effect that may have been caused by the medicine. The term 'suspect medicine' should be interpreted to mean a medicine that is thought to have caused an adverse effect.

Further details of the adverse effect, such as the time period during which the adverse effect was experienced, the severity of the side effect and the outcome of the side effect may be submitted. Further details regarding the suspect medicine may be submitted, for example the time period during which the suspect medicine was taken, the dosage regime and the medical indication of the suspect medicine, i.e. the medical condition for which the medicine was taken to treat. Information regarding any other medicines that a patient may have taken may also be input. Information regarding medical investigation may be submitted. For example, the results of a previous medical test may be input. A summary of the information may then be displayed to the user for confirmation. The information may then be converted into a format readable by a client and exported to the client. As used herein the term 'client' signifies the body to whom the information is submitted. Examples of clients include pharmaceutical companies, Regulatory Authorities, cosmetics manufacturers, food and drink companies and so forth. The questions displayed to a user may be customised by the client so that information useful to the client may be elicited from the user.

The application may present the pages to the user in a series which constitutes a 'journey'. Certain pages with requests for information thereon may be displayed in response to information provided by the user earlier in the journey. Information provided by the user may trigger certain questions at a later point in the journey. As such, the application provides a dynamic interface for a user to input information.

In the description of exemplary embodiments medicines and side effects thereto are mentioned. However, it should be borne in mind that the present invention may be used for reporting an adverse effect that a user may believe is attributable to other substances. Examples include, but are not limited to, medical devices, cosmetic products, foodstuffs, drinks, vaccines, contraceptives etc.

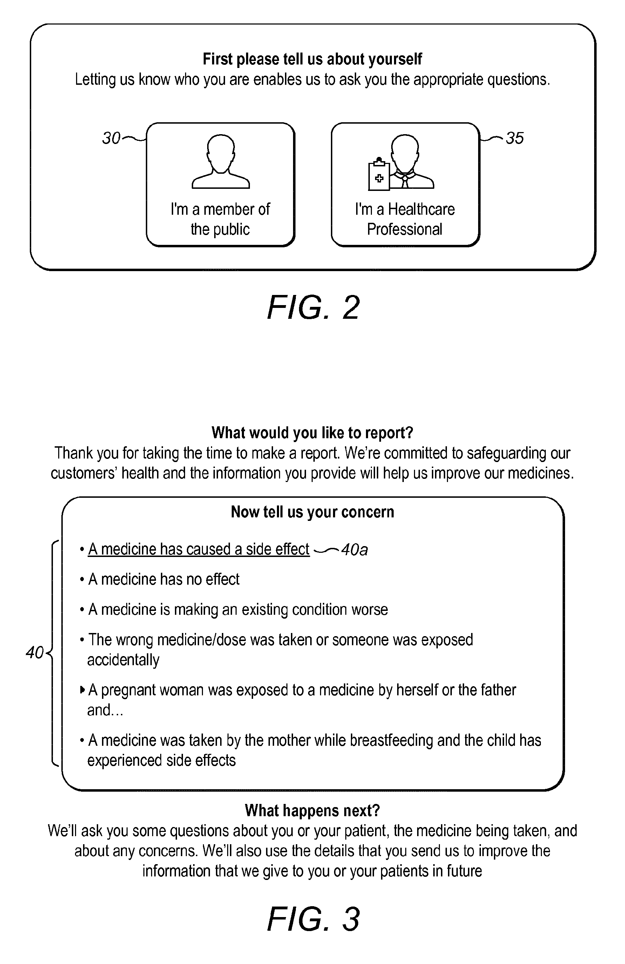

Figure 2 is a screenshot of a page displayed in the journey. The user is presented with two buttons 30, 35. The first button 30 is intended to be selected by a user who is a member of the public, i.e. a person with limited medical knowledge or layperson. The second button 35 is intended to be selected by a user who is a healthcare professional such as a doctor, nurse or dentist. As such, the journey taken by a user through the process varies depending on whether the user is a healthcare professional or not. As will be described later, certain information is requested from healthcare professionals that is not requested from laypeople. This distinction leads to a better user interface since laypeople will not be asked for information they are unlikely to be able to provide, and conversely healthcare professionals are prompted to provide information having a greater degree of detail. This distinction will also improve the reliability of the information that is exported to the client at the end of the process. In the example described herein, the user selects the healthcare professional button 35.

Figure 3 is a screenshot of an adverse event scenario selection page. The user can select on of the scenarios listed on the page. In the example described herein, the user selects the scenario 40a entitled "A medicine has caused a side effect". That is to say that the adverse event to be reported, in this example, is a side effect that the user suspects may have been caused by a medicine. Other scenarios include "A medicine has no effect", "A medicine is making an existing condition worse", "The wrong medicine/dose was taken", "A pregnant woman was exposed to a medicine" and "A medicine was taken by the mother while breastfeeding". However, alternative scenarios may be displayed on this page. Possible alternatives will depend on the field of the client who has commissioned the application. For example, a cosmetics company may wish to list potential scenarios related to use of one of their products. Likewise a food company may wish to list alternative scenarios and so forth. As such, it is possible for a user to report various types of adverse event.

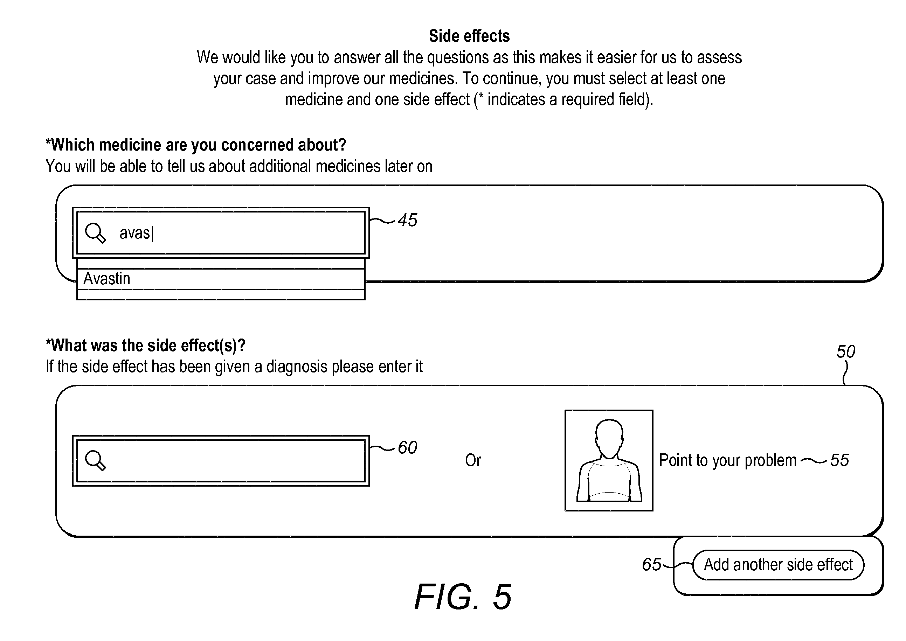

After selecting one of the scenarios 40 listed in the scenario selection page, the user may be prompted to state whether he or she agrees to certain terms and conditions. If the user does agree to the terms and conditions, the process proceeds to the side effects page shown in Figure 5. If the user does not agree to the terms and conditions, the user is returned to the scenario selection page shown in Figure 3.

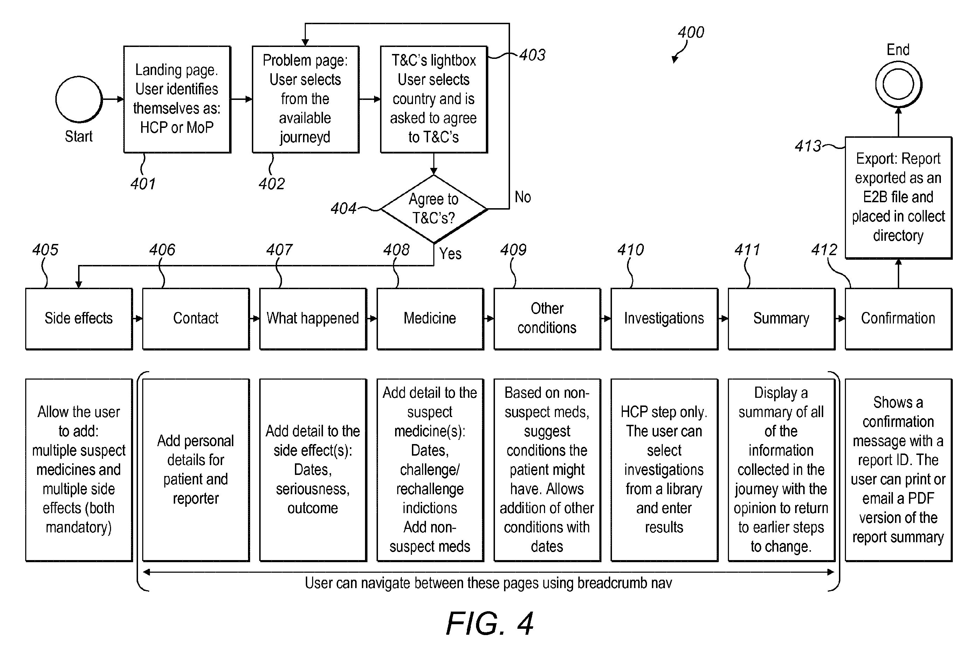

Figure 4 is a flow diagram that represents a user journey 400 experienced by the user after he or she has selected, at step 401, the healthcare professional button 35, the scenario 40a entitled "A medicine has caused a side effect" at step 402 and has agreed (at step 404) to the terms and conditions agreement request at step 403.

At step 405, the user is requested to identify a side effect. A side effect page is shown in Figure 5. The side effect page allows the user to identify the suspect medicine and one or more side effects that he or she would like to report.

A suspect medicine auto-complete field 45 is provided. A user types the name of a medicine that he or she believes may have caused a side effect. As the user begins to type the name of the medicine a dropdown menu is displayed showing suggested medicine names. The search function operates in the following manner. As the user types the name of the medicine, the search server 20 searches the database 25 and specifically a schema stored in the database 25 relating only to medicines provided by the client. Once the user sees, in the dropdown menu, the name of the medicine that is suspected of causing the side effect, he or she may select it. In this example, the user begins to type "avas". The only medicine in the schema relating to medicines provided by the client is having a similar name is Avastin (TM) and so Avastin (TM) is displayed as a suggestion in the drop down menu. The user selects Avastin (TM).

In embodiments of the invention, the application may be customised by a client, for example a pharmaceutical company. In such cases, the reporting of suspect medicines may be limited to those medicines provided by the client and exclude those medicines provided by other providers. As the user types in the name of the suspect medicine, the only those medicines provided by the client are suggested to the user in the dropdown menu. Other possible suspect medicines that are not manufactured by the client can be entered using a similar approach as above but searching is of a global medicine dictionary.

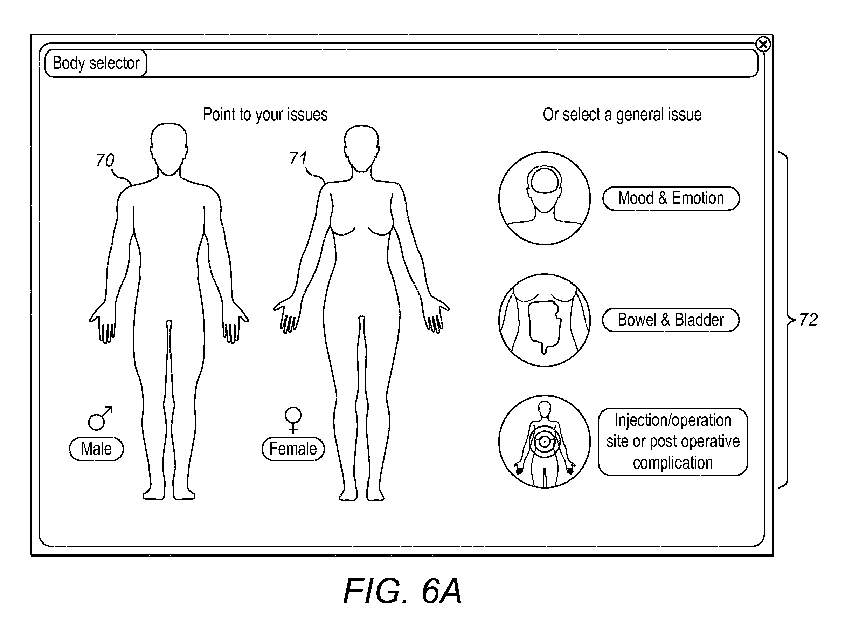

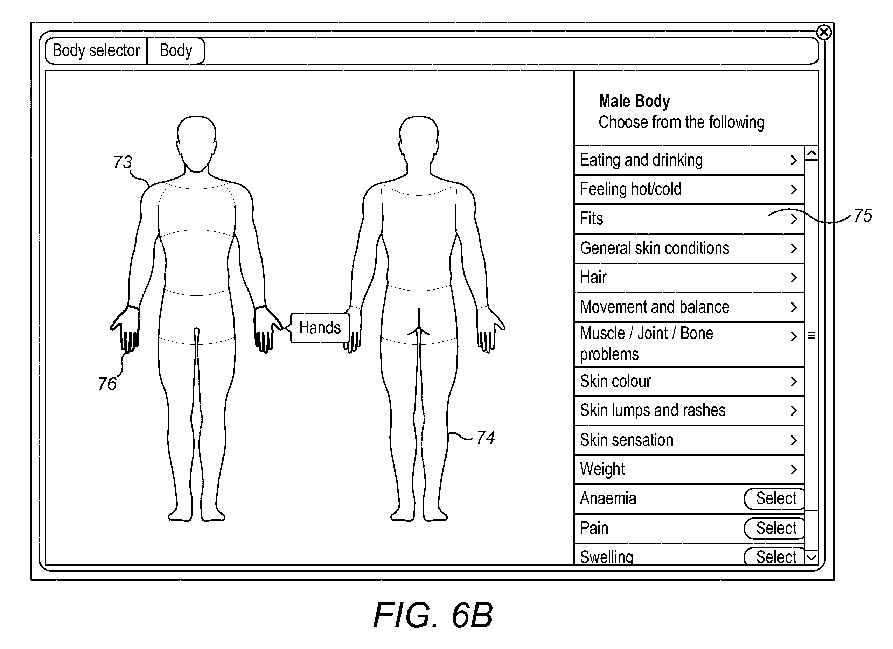

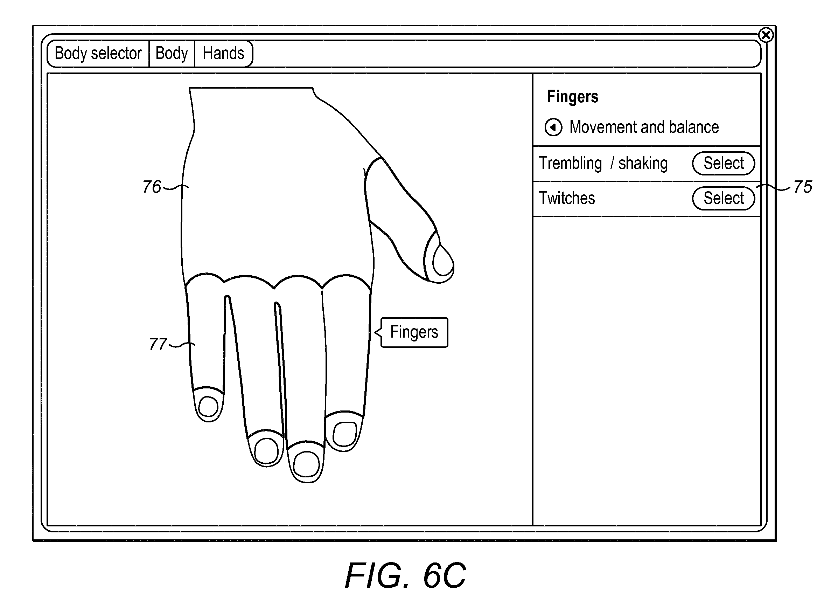

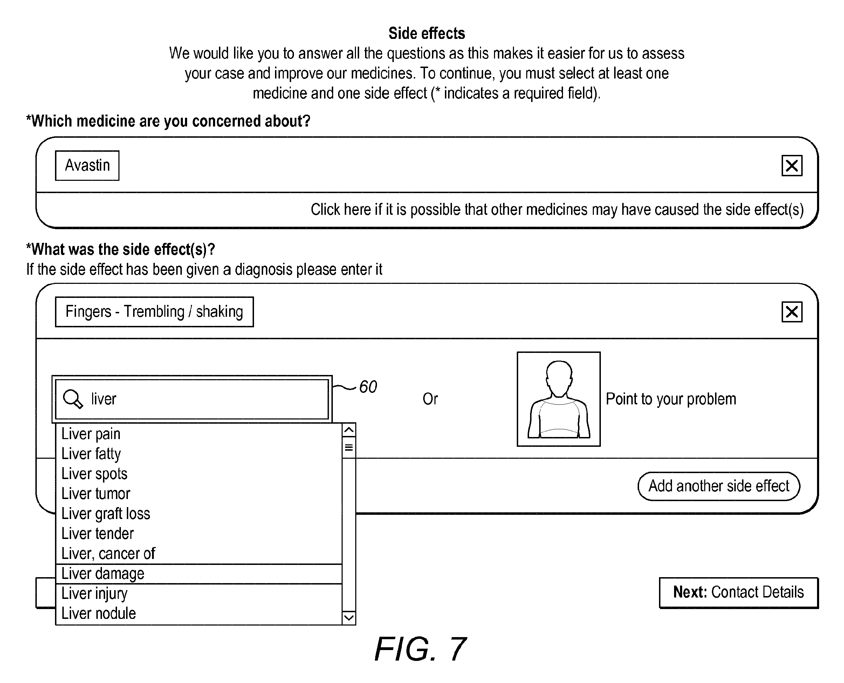

The side effect page comprises a side effect module 50. The side effect module 50 allows the patient to enter information in order to identify the side effect he or she wishes to report. The user may identify a side effect in two ways. The user may select a body map button 55. If the user selects the body map button 55, a body map page is displayed which will be described with reference to Figures 6 and 7. Alternatively, the user may type the name of the side effect in a side effect auto-complete field 60. After entering the identity of one side effect, further side effects may be identified by selecting an additional side effect button 65.

Figures 6A-C show a body map function which may be used to identify the side effect and associated physical location that the user wishes to report.

Figure 6A shows the first page seen by the user after selecting the body map button 55. The user is presented with a choice of a male body 70, a female body 71 and three general icons 72. In this example, the user selects the male body icon since the user wishes to report a side effect that has affected a male patient. If the user wished to report a side effect that has affected a female patient he or she would select the female body map. Certain general conditions, such as conditions that may be difficult to represent physically e.g. those relating to mood and emotion, bowel and bladder function or injection/operation related issues may be selected by choosing the appropriate general icon 72. The user is guided to select their reaction or event from a menu of colloquial terms. Since the application is both configurable and customisable, the "general" icons 72 may be varied to report different types of conditions.

After selecting the male body 70, a front view 73 and rear view 74 of the male body 70 are displayed, as shown in Figure 6B. Also displayed is a list of keywords 75 that may relate to a side effect of the male body that the user wishes to report. Examples of keywords may be certain headings such as "Eating and drinking", "Feeling hot/cold" etc. If a user selects one of these keywords, subheadings or specific side effects falling within the scope of the selected heading are displayed. A user may navigate through such headings and subheadings to find the appropriate side effect he or she wishes to report.

The front view 73 and the rear view 74 are each divided into body elements such as head, chest, arms, hands, abdomen, groin, back, bottom, legs and feet. The user may select the element of either the front view 73 or the rear view 74 that corresponds to the area of the body that is affected by the side effect they wish to report. Alternatively, the user may select one of the keywords 75. In the example, shown in Figure 6B, the user selects the element 76 entitled "Hands". In response, the body map zooms in to the hands 76 of the male body, as shown in Figure 6C.

The view in Figure 6C is a zoomed in view of a hand 76 of the male body 70. The list of keywords 75 is updated to list keywords relating specifically to the hands. Once the body map has zoomed in to an element of the body sub-elements thereof may be displayed. In Figure 6C, a subelement 77 entitled "Fingers" of the element 76 entitled "Hands", is selected. Upon selection of the subelement, the list of keywords 75 may, once again, be updated to list those keywords relating to possible side effects relating specifically to the fingers. These key words have been selected to represent those terms most frequently reported by patients. Where medical terms were considered too technical for patients to understand, these have been converted to a set of colloquial terms that individually map to a specific medical term contained in the dictionary Medical Dictionary for Regulatory Activities (MedDRA).

Two potential side effects relating to the subelement 77 entitled "Fingers" are displayed in this example, namely "Trembling/shaking" and "Twitches". In this example "Trembling/shaking" is selected by the user.

After selecting one side effect, the user can report further side effects by selecting the additional side effect button 65 shown in Figure 5.

Figure 7 is a screenshot illustrating a user entering text into side effect auto-complete field 60. As the user begins to type the name of the side effect he or she wishes to report suggestions are displayed in a dropdown menu below the side effect auto-complete field 60. When the user sees the identity of the side effect to be reported the side effect may be selected by the user, for example by clicking on it with a mouse. In this example, the user types the word "liver" into the side effect auto-complete field 60. Suggested side effects appear in the dropdown menu. The user selects "Liver damage" from the dropdown menu.

Possible side effects that may be reported are stored in the database 25. The terms that appear in the dropdown menu may be recognised medical terms or more colloquial terms equivalent to recognised medical terms. Recognised medical terms for side effects may be derived from a recognised medical classification dictionary such as the Medical Dictionary for Regulatory Activities (MedDRA). Such terms may be organised in a MedDRA schema in the database 25 so that they may be searched quickly by the search server 20. In addition, searching by the reporter is optimised so that most frequently reported terms appear first (sinusitis versus sinus arrest) and terms unrelated to adverse events are filtered out (laboratory tests do not appear). The database may contain a library of colloquial terms that a layperson might use in everyday speech to describe a side effect. Each colloquial term may be stored in the database 25 as an item having a tag mapping that term to a recognised medical term in the MedDRA schema. As such, a user who is not familiar with medical terminology may submit a report that describes a side effect to a similar degree of accuracy as a report submitted by a medical professional.

Such optimisation of the searching is also provided in respect of the list of keywords 75 that may relate to a side effect of the male body that the user wishes to report, as shown in Figure 6B.

After the user has finished selecting the side effect he or she would like to report, the process 400 continues to step 406 where user and patient personal details may be submitted. Details submitted may include name, date of birth, address, email address, height, weight and gender of the user. If the user is a medical professional then fields may be provided for contact details of the medical professional as well as for the patient's details.

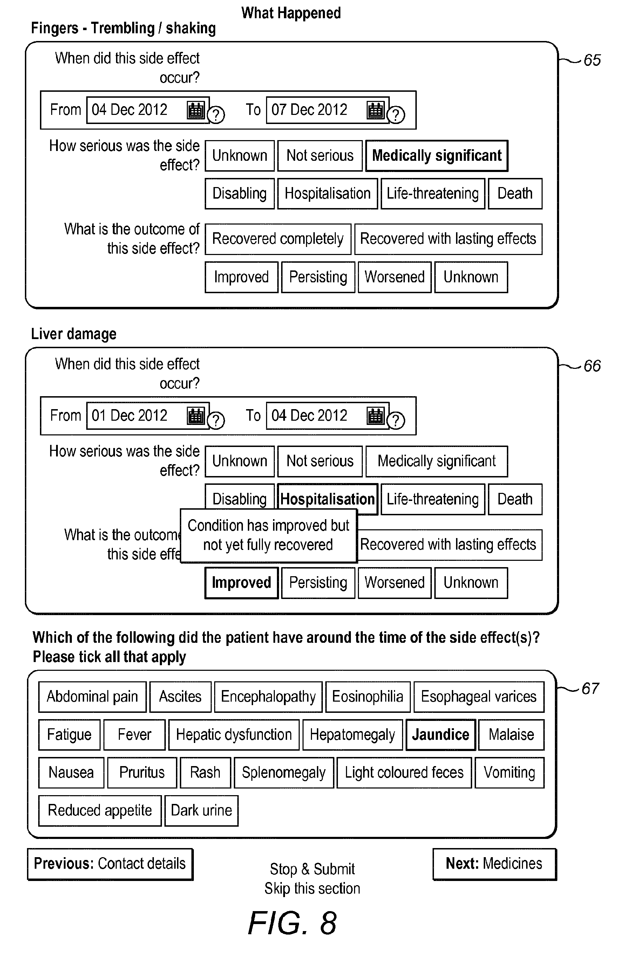

At step 407 further details relating to the side effect to be reported may be submitted. Figure 8 is a screenshot of a "What Happened?" page. A side effect module appears for each side effect selected at step 405. In this example, a "Fingers - Trembling/shaking" module 65 and a "Liver damage" module 66 are displayed.

Each side effect module contains queries regarding details of the side effect. For example, dates may be entered to signify a period during which the side effect was experienced. Questions directed towards the seriousness of the side effect and to the outcome of the side effect may be presented to the user. The user may be prompted to select an answer from multiple possible answers displayed to the user, as shown in Figure 8.

Also displayed in the "What Happened?" page is a triggered question 67. When the user is a healthcare professional, they are asked to report any additional symptoms related to a side effect. In this case, the selection of liver damage as a side effect triggers a question asking the user to identify symptoms which may be associated with liver damage. For example, symptoms such as abdominal pain, fatigue, hepatomegaly and jaundice, amongst others, are displayed as possible symptoms. The user is invited to select any additional symptoms. In this example, the user selects "Jaundice".

The triggering of certain questions may be controlled by the software stored in the application server 15. Once a particular side effect is identified at step 405, a the application checks to see whether the identified side effect has any triggered requests for associated information associated therewith. Additional questions can be triggered when either a particular side effect is entered or when a combination of a medicine and a side effect is entered. The application is configurable, which enables clients such as pharmaceutical companies to provide lists of triggered questions for products. These are automatically provided to a user when they enter a particular medicine and side effect. The lists can be provided discreetly.

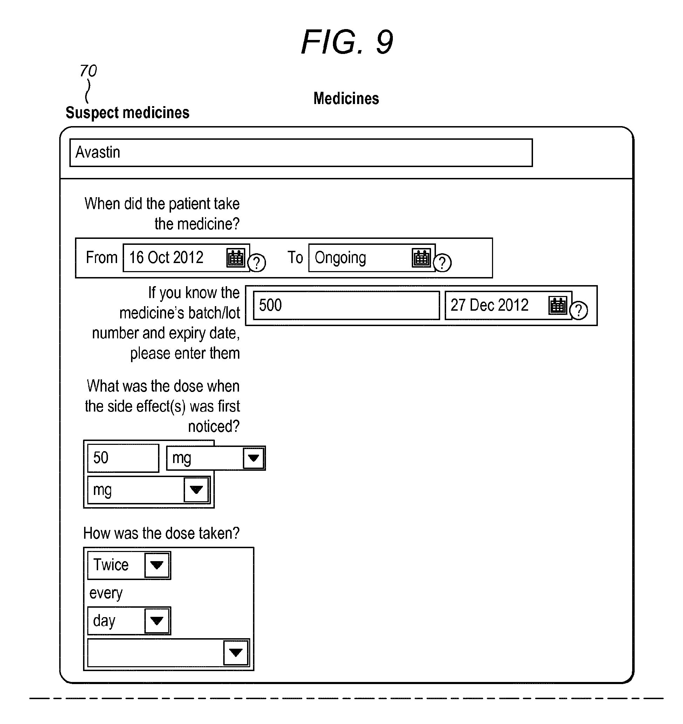

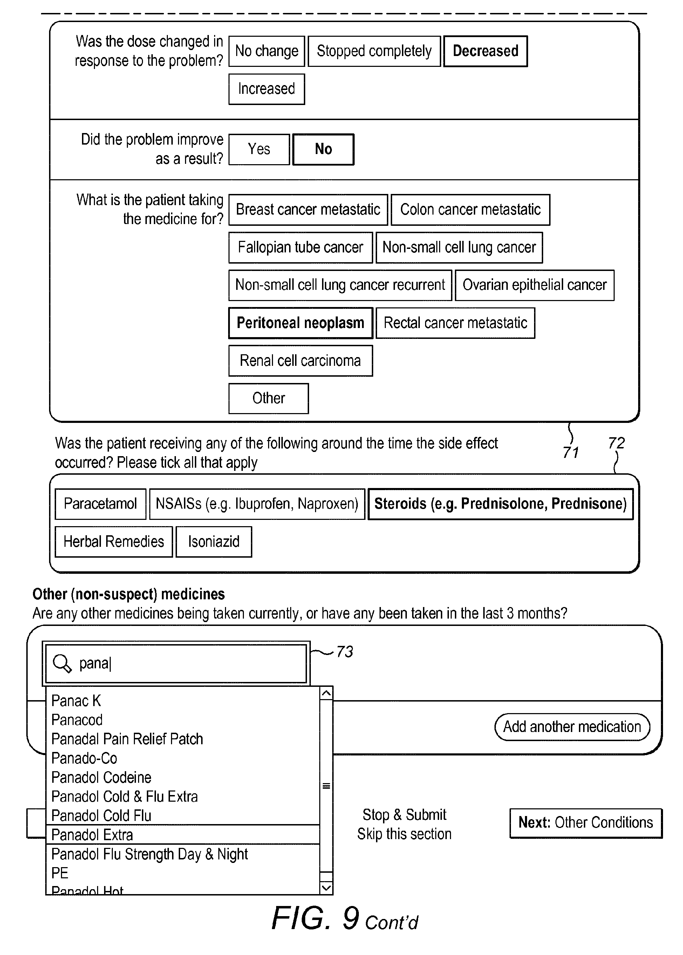

After entering further details relating to the side effect at step 407, the process 400 proceeds to step 408. Figure 9 is a screenshot of a "Medicines" page. The page shown in Figure 9 has a suspect medicines module 70. The suspect medicines module 70 comprises questions and entry fields for providing further information regarding the suspect medicine.

Information that may be submitted includes the time period during which the suspect medicine was taken, the batch number and expiry date of the medicine, details of the dosage regime. Appropriate entry fields may be provided depending on the information requested in a particular question. For example, dates may be entered using a drop down calendar of a type known in the art. When entering a value for a dose of medicine, the user may be prompted to select a unit (such as grams, mg, percentage) from a drop down menu. Requesting information be entered using such drop down menus helps to ensure that information is submitted in a format suited to and defined by the client.

The suspect medicines module 70 comprises a query 71 requesting that the user select an indication of the suspect medicine. The term 'indication' should be understood to mean a reason to use a certain test, medication, procedure, or surgery. In this example, the user has identified Avastin (TM) as a suspect medicine. Possible indications associated with Avastin (TM) are stored in the database 25 and are retrieved upon selection of Avastin (TM) by the user. The possible indications are displayed to the user as a multiple choice question. This reduces the possibility of a user accidentally entering an indication that is not usually associated with a selected medicine. Use of the medicine for other unlisted indications can be provided through an autocomplete selection through intelligent searching of the MedDRA library.

A query 72 is shown which is triggered by the identity of the side effect. The user is asked whether the patient received any of a multiple choice of other medicines at the time the side effect was experienced. Whether the user is asked this question, and the multiple possible answers that are displayed is dependent on the identity of the side effect, as determined at step 405 of the process 400. In this example, entering "Liver damage" as a side effect prompts the application to ask the question and to give options of paracetemol, NSAIDs, steroids, herbal remedies and isoniazid. In alternative embodiments, the wording of the question may differ and the choices presented to the user may differ.

A non-suspect medicine field 73 is also provided on the Medicines page shown in Figure 9. A user is asked to enter the name of any other medicines that the patient has taken. As the user types the name of a medicine the search server 20 searches the database 25 for medicines to suggest and suggested names are displayed in a drop down menu. However, the search is not limited to schemas containing client medicines. As such, medicines from a variety of providers may be suggested. In this example, the user selects Panadol Extra (TM) from the drop down menu after typing "pana" into the non-suspect medicine field 73.

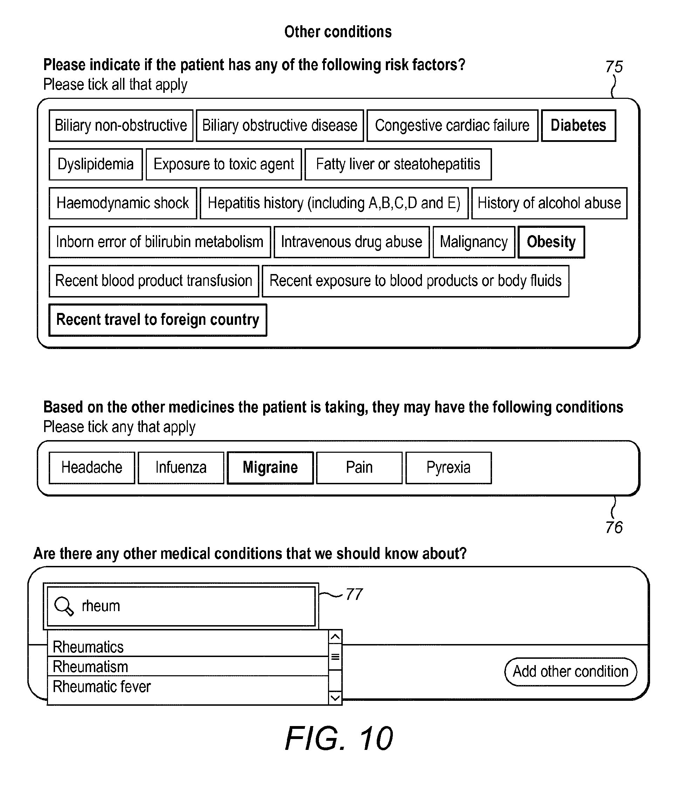

Figure 10 is a screenshot of a module of questions that allow a user to enter information about any other conditions the patient may experience. A question 75 is triggered by the user selection of "liver damage" as a side effect. The question asks the user to select any applicable 'risk factors' from several displayed to the user. In other embodiments, a different question may be asked and different selectable answers displayed depending on the identity of the side effect selected by the user. For example, a user reporting heart disease as a side effect may be offered a different choice of 'risk factors' compared with someone entering headaches as a side effect.

A question 76 that is triggered by the identity of a non-suspect medicine identified at step 408 is also displayed in Figure 10. The user is asked to identify an indication of the non-suspect medicine identified at step 408. In this example, since the user entered Panadol Extra (TM) as a non-suspect medicine possible indications displayed to the user are headache, influenza, migraine, pain, pyrexia. In this example, the user selects "migraine".

Also provided on the "Other Conditions" shown in Figure 10 is an other medical condition auto-complete field 77. The user may begin to type the name of another condition experienced by the patient. The schema stored in the database relating to medical conditions is searched by the search server 20 and suggestions displayed in a drop down menu. In this example, the user types "rheum" and selects "Rheumatism" from the suggested conditions displayed.

After information relating to other conditions the process moves on to step 410 where information relating to investigations performed on the patient may be entered. In certain embodiments, this information is only requested from users that identify themselves as healthcare professionals at step 401.

A triggered module 80 of questions may be displayed in response to a user entering a specific side effect at step 405. In this example, since liver damage was entered as a side effect, a module of questions regarding tests that may be performed in the case of liver damage is displayed. The questions displayed to the user will vary depending on the side effect entered at step 405. For example, questions requesting information on tests performed on a patient reporting kidney damage as a side effect will differ from those requesting information on tests performed on a patient reporting migraines as a side effect.

The user can enter test results in a test result field 81. A range of normal results for a particular test may be stored in the database 25. If a test result is entered in the field 81 that is outside this range, a signal 82 may be provided to the user that the entered value is abnormal. A healthcare professional user may amend the boundaries of the normal ranges if necessary to align with their local laboratory facility.

An investigation auto-complete field 85 is also provided. A user may begin to enter the name of an investigation carried out on a patient. The search server 20 searches the schema containing the identities of medical investigations. Suggested results are displayed in a drop down menu. In this example, a user begins to type "rena" and selects "Renal scan" from the suggestions shown in the drop down menu. Further questions may then be displayed asking the user to enter results of the selected investigation.

After the step 410, the process moves on to step 411. A summary of the information entered by the user is displayed. The user may then review the information entered. The user may elect to change information that has been entered.

The user may navigate between pages displayed between steps 406 and 411 by selecting a link displayed in a navigation bar 90 to the page to which the user wishes to navigate. The navigation bar 90 may be displayed on each page displayed to the user during the process 400 between steps 406 and 411. The ability to navigate may be provided a navigation tool such as Breadcrumb.

Once the user has approved the summary of information entered, the user provides confirmation by selecting a confirm button at step 412. A report of the information entered by the user is produced. The report may be rendered in a format that is readable by client systems. In the medical field, E2B is the standard format. In this example, the report is rendered in E2B format and exported to the client. Export of the report may be via email or automated submission over a network such as the internet.

While the system has been described above with reference to the process 400, it should be borne in mind that alternative processes may be followed. For example, a user may select, on the scenario selection page, an adverse event scenario other than the scenario 40a entitled "A medicine has caused a side effect". For example, if the user selects the scenario entitled "A medicine is making an existing condition worse" an existing condition auto-complete field may be displayed to the user during the ensuing process so that the user can identify the existing condition that a medicine has made worse. The existing condition auto-complete field may be displayed in addition to requests for information that are substantially the same as those described above as part of the process 400.

The customisable nature of the system allows a client to add questions to the process depending on the adverse event scenario selected on the scenario selection page shown in Figure 3. Furthermore, some of the pages herein before described with reference to the process 400 may be omitted if the client considers their inclusion to be inappropriate with respect to the selected scenario. Additional or alternative questions may be included in the process. Such additional or alternative questions may be displayed in modules on additional pages. The questions may request information be input using an auto-complete field or selection from a multiple choice of responses displayed to the user.

If a user selects the scenario "The wrong medicine/dose was taken or someone was exposed accidentally" then, in the next screen, a multiple choice of potential accidents may be displayed to the user from which the user may select one. Follow-up questions may then be triggered as part of the process. For example, if the user may select an option stating that a medicine has been taken by the wrong person. Questions relating to the time period and dose taken may then be triggered.

As such, the adverse event scenario page allows the user to identify various types of adverse event.

The system described above can provides a interface for use by patients or healthcare professional enrolled in a clinical study program to enter data through a (if desired, secure) application tailored to the investigational product or products in question. This can simplify and speed up data entry through fields configured to the particular study (e.g. patient, health centre/hospital, physician, medications). Here, the graphical interface can be used to understand the location or distribution of patient signs and symptoms when these patients are part of a clinical research study. For instance patients can highlight the location of skin lesions pre- and post-treatment with a medication enabling accurate understanding of the clinical outcome.

Embodiments of the system described above may include the capability to import identification metadata regarding, for example: clinical study identifiers, investigator identifiers and patient identifiers. The study metadata is imported directly from client lists of doctors, health centres and patients participating in the study. Importing metadata in this way removes the need for duplicate data entry reducing errors and improving the usability of the system.

Various embodiments also enable adverse event reports to be made by investigators, such as healthcare professionals, and patients who are registered to the system and for these reports to be followed up within the application in a secure manner with user authentication. The registered healthcare professional may provide follow up data directly into the system. Alternatively the system may through an automated alert process trigger requests for follow-up information to healthcare professionals. This follow up information is entered directly into the system and is appended to the original case report.

Reports can be exported as an XML file in standard format (ICH E2B) direct into a client database from which they can be reported direct to Regulatory authorities, compiled into an aggregate report or evaluated to assess the benefit risk profile of the product. In addition the reports may be provided in an appropriate readable form into separate clinical data management systems.

As an alternative or in addition to reporting adverse events, embodiments may be used for long-term study of patients to record an outcome of an approved medicine. Such a system may be configured to capture data on both the associated benefits and associated risks of using the particular medication.

Information that may be gathered in this way may include patient reported outcomes, such as an improvement in a medical condition or biometric data such as blood pressure changes, blood glucose levels. Patients may be reminded to provide defined information through reminders through mobile or devices or computers.

Information gathered may also include information about persisting or newly arising adverse events; alerts based on changes in biometric data (for a example a significant fall in blood pressure) or a deleterious change in patient reported outcomes.

Such patient reported outcomes may be entered by the user according to a process substantially similar to that described above with respect to reporting adverse events. It will be understood, that the wording of questions and information presented to the user is customisable to allow for this alternative embodiment.

Furthermore, a body map may be presented to the user to allow outcomes to be reported easily in a manner substantially similar to the body map function described above. Additionally, a suitable medical library such as MedDRA may be utilised to allow patient entered colloquial terms to be accurately mapped to corresponding recognised medical terms.

Embodiments can incorporate user authentication and be configured to receive metadata from other electronic data capture systems thereby reducing errors and simplifying the user experience through avoidance of duplicate data entry. Embodiments of the system described above may include the capability to import identification metadata regarding, for example: clinical study identifiers, investigator identifiers and patient identifiers. The study metadata is imported directly from client lists of doctors, health centres and patients participating in the study. Reports can be exported as an XML file in E2B format direct into a safety database or provided in an appropriate readable form into separate clinical data management systems.

The system, according to various embodiments, may be configured to operate on a range of mobile platforms.

Embodiments that run on a mobile platform can be configured to operate on a mobile device such as a smartphone or tablet as an application specific to that device.

As well as running embodiments that are used to report adverse events, embodiments used to report patient outcomes that can be integrated into a clinical study or be part of post-market patient support program may be supported on a mobile platform. Such a system operating on a mobile platform may be configured to capture data on both the associated benefits and associated risks of using a particular medication.

Outcomes that relate to benefits include patient reported outcomes and biometric data such as blood pressure changes, blood glucose levels etc.

Outcomes that relate to risks include adverse events (as described above); alerts based on changes in biometric data (e.g. a significant fall in blood pressure) or a deleterious change in patient reported outcomes.

The system can be pre-configured to collect defined data from a patient taking a named product, deliver reminders, trigger questions dependent on the data provided and segregate the export of the data to a client or medical staff managing the patient. A mobile platform may include user authentication and may link to a dashboard of data entered into the system via the same or another platform, for example a desktop platform.

In a post-market setting, the system can be pre-configured to collect defined data on a single specified product and trigger questions dependent on the data provided. This enables rapid capture of relevant data, reduces errors and improves usability of the application.

Various advantages are provided by the above-described system.
- Firstly, users are guided to make user inputs in such a way that the number of possible user inputs is greatly reduced compared to the alternative in which free text entry is permitted. In the case of inputs relating to side effects, for instance, users are directed through the auto-updating of lists and the body map features to specific side effects. This ensures that only the provided options can be selected, although this is done in such a way that users can easily find the option that is appropriate to them. In addition by using the body map, patients are able to provide accurate information on the localisation of their reactions or events. These features of the system thus provide a better user interface.
- Secondly, the displaying to the user of at least one further request for information, wherein the content of the request displayed to the user is dependent on previously entered information, can simplify the data entry process for the user, and can also result in more complete entry of relevant data. Data entry can be simplified because the provision of the further request with relevant content results in the user needing to perform less searching for the data that they wish to enter. Data entry can be more complete because the user is provided with options for entering further information that they might otherwise have not known or not understood was relevant to the data reporting. This may eliminate the need for current manual "follow up" processes that can be needed to fill gaps in the initial data provided.
- Thirdly, allowing selection only of provided options, e.g. for side effects, results in the collected data being of higher quality compared to alternative data collection schemas. The data is higher quality because the data is consistent between different patients, on the basis that effects experienced by different patients are less likely to be entered differently. This makes the processing of the data received from multiple patients more reliable, for instance allowing it to be more readily identified that the same side effect has been experienced by different patients. Moreover, it makes the collected data more susceptible to processing in a fully automated, or at least more automated, manner than is possible with current data collection systems.
- Rapid and accurate identification of a medicine of interest is associated with three features, namely the provision to a non-expert user with a graphical interface that enables selection of reactions or events using a body image, the tailoring of an initial selection of medicines to a particular client (e.g. pharmaceutical company), and the performance of selection through an auto-updating list.
- Export of the data in a fully automated and structured electronic form (e.g. E2B) helps to ensure that there is no corruption or alteration of the data through manual processing steps. This can result in data that is of better quality.
- The provision of data electronically (e.g. via E2B), can provide the data on the adverse events or quality issues suffered by patients almost immediately for medical evaluation. This can speed up the identification of new safety issues with medical products.
- The products and events entered triggering immediate feedback to the reporter on how to optimise management of the reported issues, can provide a patient with real time risk management In the sense that patients can be equipped to use their medicines more effectively with accessible information in line with the license.
- Coordinated capture of adverse reactions or events patients experience and issues identified can be enabled simultaneously with the quality of products (manufacturing issues, counterfeits etc....).
- The capture of reactions or events that may affect not only a mother but also a baby exposed to medicines while the mother is pregnant is enabled. This may be important given the need identify any risks to an unborn child and the potentially relatively long time frame between exposure to the medicine and the delivery of a child.
- Features of the embodiments also provide an online route for some users, particularly physicians, to rapidly and simply enter additional follow-up data on a patient who has already reported reactions or events. Moreover, this can be achieved securely.
- The complete, consistent and coded data enables the development of a datamart for purposes of performing analytics and data mining to identify safety issues related to medicinal or any other products in question.

It will be appreciated that the above described embodiments are purely illustrative and are not limiting on the scope of the invention. Other variations and modifications will be apparent to persons skilled in the art upon reading the present application. Moreover, the disclosure of the present application should be understood to include any novel features or any novel combination of features either explicitly or implicitly disclosed herein or any generalisation thereof and during the prosecution of the present application or of any application derived therefrom, new claims may be formulated to cover any such features and/or combination of such features.

## Claims

1. A computer implemented method of collecting data relating to an adverse event relating to use of a substance, the method comprising:
detecting a user input relating to an identity of the substance or to an identity of the adverse event;
determining the identity of the substance or of the adverse event based on the user input;
selectively displaying to the user at least one further request for information,
wherein the content of the request displayed to the user is dependent on the identity of the substance or of the adverse event, and
generating a report containing data collected from the user.

2. A method according to claim 1, wherein
a) the user input comprises at least partial completion of an auto-complete text field requesting the identity of the substance or adverse event, the method comprising the auto-complete text field displaying at least one potential substance or adverse event, for instance in a drop down menu; or
b) a request for user input relating to an identity of the adverse event is displayed and the request comprises displaying a body map and information relating to at least one potential adverse effect associated with the body map or a part thereof.

3. A method according to claim 2, wherein determining an identity of the adverse effect comprises detecting a user selection of a part of the displayed body map and a user selection of information relating to an adverse effect from the at least one displayed potential adverse effect associated with the selected part of the body map.

4. A method according to either claim 2 or 3, wherein the at least one potential adverse effect is displayed as a recognised medical term or as a colloquial term associated with a recognised medical term in MedDRA.

5. A method according to any preceding claim, comprising requesting a further user input comprising at least partial completion of an auto-complete text field requesting the identity of one of either a medical condition or a medical investigation, comprising the auto-complete text field displaying, respectively, at least one potential medical condition or medical investigation in a drop down menu, and optionally comprising
a) the auto-complete text field displaying, respectively, the at least one potential medical condition or medical investigation in the drop down menu with most frequently reported terms appearing first, and optionally updating a record of frequencies at which terms are reported;
and/or
b) the auto-complete text field displaying, respectively, the at least one potential medical condition or medical investigation in the drop down menu with terms unrelated to adverse events filtered out.

6. A method as claimed in any preceding claim, further comprising displaying a list of selectable potential adverse event scenarios to the user; and/or
detecting a user input relating to an identity of a substance;
determining the identity of the substance based on the user input;
selectively displaying to the user at least one potential medical indication of the identified substance based on the identity of the substance; and
detecting a second user input to select the indication of the substance from the at least one displayed potential indication.

7. A method according to claim 6, wherein either the user input comprises at least partial completion of an auto-complete text field requesting the identity of the substance, and wherein the auto-complete text field is configured to display at least one potential substance, for instance in a drop down menu; or the method further comprises:
detecting a user input relating to an identity of an adverse event;
determining the identity of the adverse event based on the user input; and
selectively displaying to the user at least one further request for information,
wherein the content of the request displayed to the user is dependent on the identity of the substance or of the adverse event.

8. A method according to claim 7, wherein a request for user input relating to an identity of the adverse event is displayed and the request comprises displaying a body map and information relating to at least one potential adverse effect associated with the body map or a part thereof, or wherein determining an identity of the adverse effect comprises detecting a user selection of a part of the displayed body map and a user selection of information relating to an adverse effect from the at least one displayed potential adverse effect associated with the selected part of the body map.

9. A method according to either claim 7 or 8, wherein the at least one potential adverse effect is displayed as a recognised medical term or as a colloquial term associated with a recognised medical term coded in MedDRA.

10. A method according to any of claims 6 to 9, wherein a further user input is requested comprising at least partial completion of an auto-complete text field requesting the identity of one of either a medical condition or a medical investigation, comprising the auto-complete text field displaying, respectively, at least one potential medical condition or medical investigation in a drop down menu;, and optionally further comprising:
a) the auto-complete text field displaying, respectively, the at least one potential medical condition in the drop down menu with each of the product indications represented as a coded MedDRA term; and/or
b) the auto-complete text field displaying, respectively, the at least one potential medical condition in the drop down menu represented in colloquial language that is linked to a medical term in MedDRA.

11. A method as claimed in any preceding claim, further comprising exporting collected data in a structured format to a client database; and/or responding to detection of entry of one of a list of predetermined specific product names and/or events by providing a response comprising feedback or one or more additional questions.

12. A computer implemented method of collecting information relating to an adverse event, the method comprising:
displaying an interactive map representing a human body comprising a plurality of selectable elements;
detecting a user selection of one of the selectable elements;
in response to the user selection, displaying at least one potential adverse effect associated with the selected element; and
detecting a user selection of the or one of the displayed potential adverse effects.

13. A method according to claim 12, further comprising:
displaying an exploded view of the selected element in response to the user selection, the exploded view of the selected element comprising a plurality of selectable sub-elements;
detecting a user selection of one of the selectable sub-elements;
in response to the user selection, displaying at least one potential adverse effect associated with the selected subelement; and
detecting a user selection of the or one of the displayed potential adverse effects associated with the selected subelement.

14. A computer program comprising computer-readable code, which, when executed by computing apparatus, causes the computing apparatus to perform the method of any preceding claim.

15. Apparatus configured to perform the method of any of claims 1 to 13.
